(19) Europäisches Patentamt
European Patent Office
Office européen des brevets

(11) Publication number: **0 459 666 B1**

(12) # EUROPEAN PATENT SPECIFICATION

(45) Date of publication of patent specification: **09.11.94** (51) Int. Cl.$^{5}$: **A61K 31/47**

(21) Application number: **91304430.1**

(22) Date of filing: **17.05.91**

(54) **Medicaments against impotence.**

(30) Priority: **31.05.90 US 531494**

(43) Date of publication of application:
**04.12.91 Bulletin 91/49**

(45) Publication of the grant of the patent:
**09.11.94 Bulletin 94/45**

(84) Designated Contracting States:
**AT BE CH DE DK FR GB GR IT LI LU NL SE**

(56) References cited:
**US-A- 4 925 837**

**AM. J. HYPERTENSION, vol. 2, no. 9, September 1989, pages 729-735, The American Journal of Hypertension, Inc.; R.R. LUTHER: "New perspectives on selective alpha1 blockade"**

**EUROPEAN JOURNAL OF PHARMACOLOGY, vol. 186, September 1990, pages 87-93, Elsevier Science Publishers B.V. (Biomedical Division); F. HOLMOUIST et al.: "Effect of the alpha1-adrenoceptor antagonist R-(-)-YM12617 on isolated human penile erectile tissue and vas deferens"**

**J. AUTONOM. PHARMACOL., vol. 5, 1985, pages 81-88; H. HEDLUND et al.: "Comparison of the responses to drugs acting on adrenoreceptors and muscarinic receptors in human isolated corpus cavernosum and cavernous artery"**

**Br. J. Pharmacol. 1988, 95(4), p. 1241-54**

(73) Proprietor: **PFIZER INC.**
**235 East 42nd Street**
**New York, N.Y. 10017 (US)**

(72) Inventor: **Milne, George McLean**
**24 Bishop Bay Drive**
**Niantic, Connecticut (US)**
Inventor: **Wyllie, Michael Grant**
**14 Stanmore Court**
**Canterbury, Kent (GB)**

(74) Representative: **Bradbrook, Geoffrey William et al**
**PFIZER LIMITED**
**Ramsgate Road**
**Sandwich Kent, CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The field of art to which this invention pertains is the treatment of male erectile impotence and more particularly the use of erection enhancing compounds to treat male erectile impotence.

Impotence is the inability to obtain and sustain an erection sufficient for intercourse. The penis becomes erect when certain tissues (in particular, the corpora cavernosa) in the central portion of the penis become engorged with blood thereby causing them to become less flaccid, and in turn causing an erection. It is estimated that 10-12 million American men between the ages of 18 and 75 suffer from chronic impotence with the great majority being over the age of 55.

Impotence results from psychologic disturbances (psychogenic), from physiologic abnormalities (organic) or from a combination of both. Typically, multiple factors are responsible for impotence.

The major causes of organic impotence are vascular abnormalities, neurologic deficiencies and drug treatment side effects. The primary vascular causes of impotence are arterial insufficiency, which prevents the penis from filling with blood, and venous abnormalities that prevent the retention of blood in the penis during the erectile process. Arterial insufficiency is primarily due to atherosclerosis and has been found to be exacerbated by smoking. A less frequent and somewhat unlikely vascular cause of impotence is priapism, prolonged painful erection, which can cause hypoxia and death of penile tissue.

The search for impotence treatment methods has included external devices for example, tourniquets (see U.S. Patent No. 2,818,855). In addition penile implants, such as hinged or solids rods and inflatable, spring driven or hydraulic models, have been used for some time. The administration of erection effecting and enhancing drugs is taught in U.S. Patent No. 4,127,118. That patent teaches a method of treating male impotence by injecting into the penis an appropriate vasodilator, in particular, an adrenergic blocking agent or a smooth muscle relaxant to effect and enhance an erection. More recently U.S. Patent No. 4,801,587 teaches the application of an ointment to relieve impotence. The ointment consists of the vasodilators papaverine, hydralazine, sodium nitroprusside, phenoxybenzamine, or phentolamine and a carrier to assist absorption of the primary agent through the skin. In addition, Yohimbine, an alpha adrenergic antagonist is currently marketed with the suggestion that it may be efficacious in treating psychogenic impotence.

Although the injection of such agents as papaverine into the corpora cavernosa has proven effective, typically large doses 8 to 32 mg are required, resulting in undesired side effects such as pain, priapism and tissue fibrosis. The other impotence relieving agents also have one or more of the above problems when administered intracavernosally.

J. Autonom. Pharmacol. 1985, 5, 81-83 describes the action of drugs interacting with adreno- and muscarinic receptors on penile tissue. Br. J. Pharmacol. 1988, 95(4), 1241-54 describes the action of UK 52046 on cardiac arrhythmias.

Thus, there is a continuing search in this field of art for improved methods for relieving male erectile impotence.

The invention comprises use of compound UK-52046 or a pharmaceutically acceptable acid addition salt thereof for obtaining a medicine intended for relieving erectile impotence in a human male.

Compound U.K.-52,046, which has the chemical structure

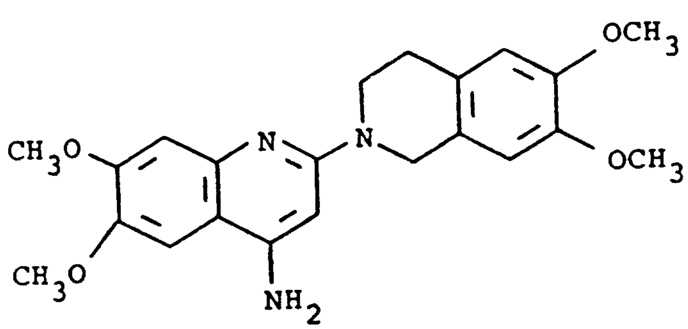

and its pharmaceutically acceptable acid addition salts are described in U.S. Patent No. 4,686,228, entitled "Quinoline Therapeutic Agents".

Although the generic name

U.K. 52,046 represent the free base, the present invention is also meant to embrace the pharmaceutically acceptable acid addition salts, such as hydrochloride, hydrobromide, hydroiodide, nitrate, sulfate, bisulfate, phosphate, acid phosphate, acetate, lactate, citrate, acid citrate, tartrate, bitartrate, salicylate,

succinate, maleate, gluconate, methane sulfate, ethane sulfate, methane sulfonate (mesylate), benzenesulfonate (besylate), toluene sulfonate and p-toluenesulfonate salts. However preferred are the mesylate or besylate salts.

Any method of administration of the above compound found effective in relieving male erectile impotence may be used. Preferably the administration is parenteral (e.g. injection, air gun) or transdermal (e.g. iontophoresis, passive transport) as these methods direct the compound to the critical tissues.

For purposes of parenteral administration, (e.g. intracavernosal) solutions of the compound in sesame or peanut oil or in aqueous propylene glycol may be employed, as well as sterile aqueous solutions of the corresponding water soluble acid addition salts previously enumerated. Such aqueous solutions Should be suitably buffered if necessary. These particular aqueous solutions are especially suitable for intracavernosal injection purposes. In this connection, the sterile aqueous media employed are readily obtained by standard techniques well known to those skilled in the art. For instance, distilled water is ordinarily used as the liquid diluent and the final preparation is passed through a suitable bacterial filter such as a sintered glass filter or a diatomaceous-earth or unglazed porcelain filter. Preferred filters of this type include the Berkefeld, the Chamberland and the Asbestos Disk-Metal Seitz filter, wherein the fluid is sucked into a sterile container with the aid of a suction pump. Needless to say, the necessary steps should be taken throughout the preparation of these injectable solutions to insure that the final products are obtained in a sterile condition. The injection can be made by needle or air gun. Typically the injection is made into the corpus cavernosum although any injection that is effective in relieving impotences may be used.

Any amount of the above described compound when administered by injection that is effective in relieving male erectile impotence may be used. Because the compound is vasodilator the drug can reduce the blood pressure resulting in systemic cardiovascular effects (e.g. fainting). Thus, preferably the amount is below that amount which is the threshold limit for a significant systemic cardiovascular effect.

The frequency of delivery, of the amounts described below, relates to the frequency of impotence relief. Typically the compound is administered just prior to the desire to obtain and sustain an erection. However as previously stated the total amount delivered is preferably below the threshold limit for significant systemic cardiovascular effects.

Preferably 0.1 µg to 25 µg U.K.-52,046 is used in a single dose.

Below the lower limit the compound is not effective and above the upper limit significant systemic cardiovascular effects may occur.

The above described compound can also be administered transdermally. For purposes of transdermal administration, the dosage from of the particular compound may include, by way of example, solutions, lotions, ointments, creams, gels, suppositories, rate-limiting sustained release formulations and devices therefor. Such dosage forms comprise the particular compound and may include ethanol, water, penetration enhancer and inert carriers such as gel-producing materials, mineral oil, emulsifying agents, benzyl alcohol and the like.

A specific transdermal flux composition comprises a pharmacologically active compound or a prodrug thereof, an aqueous ethanol solvent containing from 15% to 75% ethanol by volume and <u>cis</u>-olefin compounds such as oleic acid.

For transdermal administration the compound may, for example, simply be applied directly to the penis. In another method of transdermal application the compound may be delivered transdermally utilizing iontophoresis. In iontophoresis a drug is transported from a solution (disposed against the skin) across the dermal barrier by the application of a current across the solution and skin.

Any amount of the above described compound when administered transdermally that is effective in relieving male erectile impotence may be used.

Typically the same amounts are preferably delivered to the critical tissues as are delivered by injection, however the amounts applied transdermally are typically greater because of the low effectiveness of transdermal administration for delivery to the critical tissues. As with injection, the amounts delivered are preferably below the threshold limit for significant systemic cardiovascular effects. The frequency of delivery is the same as described for injection. The amounts detailed below are for passive transdermal delivery. It is believed that for iontophoretic delivery the ranges would be reduced by a factor of 10 based on the greater efficiency of iontophoretic delivery.

Thus, preferably 100 µg to 100 mg U.K.-52,046 is used in a single dose.

<u>EXAMPLE 1</u>

Seventeen mature adult male monkeys (age unknown) either Cercopithecus aethiops (green monkey) or Macaca fasciculata (cynomologous) weight range 4.7 to 7.1 kg were used. Animals were anaesthetised with

diazepam (2.5 mg), ketamine chloride (20 µg/kg i.m. supplemented as appropriate) and given the appropriate drug dissolved in saline intracavernosally (0.3 ml). In a separate series of experiments to determine threshold effects, varying volumes of i.v. injectable solution were injected intracavernosally. Animals were placed supine, the penis stretched out and a rubber band placed around the root of the base as a tourniquet kept in place for three minutes after the injection. The solution was injected through a 27G needle into one of the corpus cavernosa and 5, 10, 25, 30, 60 and 180 minutes later tumescence (increase in volume) and rigidity of the penis was estimated visually and by palpation. To determine the threshold effect using the injectable solution a series of animals were used covering an appropriate dose range, e.g., U.K. 52,046 (0.05 to 0.3 µg). The antidote/reversal experiments involved intracavemosal administration of noradrenaline (10 µg) phenylephrine (10 µg) or clonidine (15 µg).

Table 1 below details the comparative effect of U.K.-52,046, Doxazosin, Amlodipine, Papaverine and Phentolamine by injection to monkeys as described in Example 1 above. Surprisingly U.K.-52,046, Doxazosin, and Amlodipine all were effective for causing Tumescence and Rigidity at significantly lower doses than either Phentolamine or Papaverine. In fact, U.K.-52,046 was effective at doses 1000 times lower than for Phentolamine or Papaverine.

TABLE 1

| Comparative Effects In Monkeys | | | | | |
|---|---|---|---|---|---|
| Drug | Threshold Dose | Tumescence | Rigidity | Time Course | Reversible |
| U.K.-52,046 | 0.1 µg | + + + | + + + | S | S |
| Doxazosin | 100 µg | + + | + | S | S |
| Papaverine | 6.0 mg | + + | + + | S | S |
| Amlodipine | 500 µg | + + | + + | X | X |
| Phentolamine | 1.5 mg | + + | + + | S | S |

0 = no effect
\+ = minimal effect
\+ + = good effect
\+ + + = full effect
S = satisfactory
X = unsatisfactory

EXAMPLE 2

Sheets of excised human skin cut to about 350 µm thickness were mounted between two halves of a diffusion cell (Side-by-Side, Crown Glass) with the stratum corneum side toward the donor compartment which contained 3 ml of radiolabelled drug in the appropriate vehicle. The receiver compartment contained 3 ml of 40% (v/v) ethanol in an acetate buffer. Both compartments were stirred with a magnetic stirrer and maintained at 32 °C.

Samples were removed periodically from the receiver side of the diffusion cell, mixed with scintillation cocktail (Ecolume, ICN Radiochemicals) and counted for 10 minutes in a liquid scintillation counter (Tri-Carb 2000CA, Packard). Following an initial lag phase of several hours, the amount of radiolabeled drug appearing in the receiver side was linear with time for the duration of the experiment (routinely 24-48 hours). From a linear least squares analysis of these data the rate of appearance of UK-52,046 in the receiver chamber was determined. This value, when divided by the specific radioactivity of the drug in the donor solution and the area of exposed skin (1 $cm^2$), yielded the flux ($\mu g/cm^2/hr$). Samples removed from the donor chamber at the beginning and end of the experiment contained, within error, the same amount of UK-52,046. Thus, constant concentration of the permeant was maintained on the donor side throughout the experiment. Values for the delivery of UK-52,046 reflect total radioactivity; no measure of the amount of intact drug was made.

Table 2 below details the effective in vitro transport of UK-52,046 transdermally according to the procedure described in Example 2.

TABLE 2

| Transport of U.K.-52,046 across human skin from a solution containing 8 mg/ml drug as the mesylate salt in 40% (v/v) ethanol:buffer (pH 4.5) | | |
|---|---|---|
| Skin Type | Flux ($\mu g/cm^2/h$) | n |
| Thigh | 3.25 | 1 |
| Thigh | 3.53 | 1 |
| Thigh | 1.69 | 1 |
| Thigh | 0.95 | 3 |
| | -------- | |
| | x = 1.98 ± 0.96 | |
| Penis | 0.63 | 2 |

Table 3 below expands on Table 2 above by using a pretreatment to enhance the effective transdermal in vitro transport of UK-52,046. The procedure used was according to Example 2.

TABLE 3

*In vitro* transport of UK-52,046 across human cadaver skin as a function of various pre-treatments. The vehicle was 40% (v/v) ethanol:buffer (pH 4.5).

| Treatment[2] | Flux[1] ($\mu g/cm^2/h$) |
|---|---|
| No pre-treatment | 0.95 |
| Vehicle, no Oleic | 0.22 |
| Vehicle + 0.25% Oleic Acid | 1.26 |
| 100% Oleic Acid | 2.45 |

[1]All values are the mean of three replicates.

[2]Treatment was for 3 hours prior to the diffusion study. A concentration of 8 mg/ml UK-52,046 was employed.

This invention provides medicine for treating male erectile impotence by the administration of UK-52,046 to effect and enhance an erection. The compound is effective at very low dosages and thus reduces the undesired side effects of other compounds.

## Claims

1. The use of U.K.-52,046 which has the chemical structure

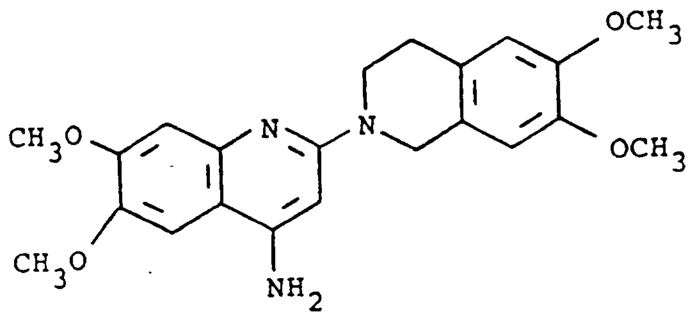

or a pharmaceutically acceptable acid addition salt thereof for obtaining a medicine intended for relieving erectile impotence in a human male.

2. The use of U.K.-52,046 or a pharmaceutically acceptable salt thereof according to claim 1 wherein the medicine is suitable for parenteral administration.

3. The use of U.K.-52,046 or a pharmaceutically acceptable salt thereof according to claim 1 wherein the medicine is suitable for transdermal administration.

4. The use as recited in claims 2 or 3 wherein the mesylate salt of U.K.-52,046 is used.

## Patentansprüche

1. Die Verwendung von U.K. 52 046, welches die nachfolgende chemische Formel

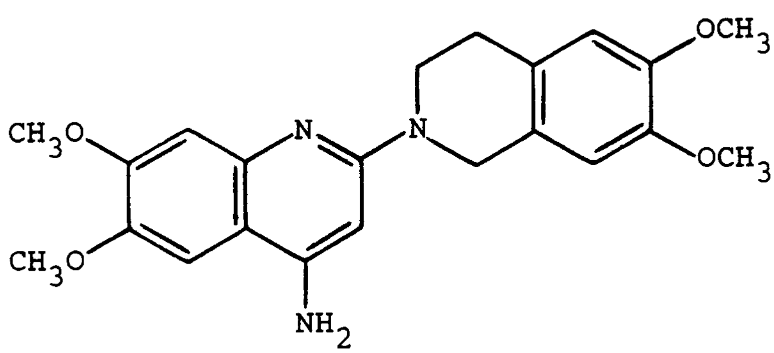

besitzt, oder eines pharmazeutisch verträglichen Säureadditionssalzes davon, zum Erhalten eines Arzneimittels zur Erleichterung der erektilen Impotenz beim Mann.

2. Die Verwendung von U.K. 52 046 oder eines pharmazeutisch verträglichen Salzes davon gemäß Anspruch 1, bei welcher das Arzneimittel für parenterale Verabreichung geeignet ist.

3. Die Verwendung von U.K. 52 046 oder eines pharmazeutisch verträglichen Salzes davon gemäß Anspruch 1, bei welcher das Arzneimittel für transdermale Verabreichung geeignet ist.

4. Die Verwendung nach den Ansprüchen 2 oder 3, bei welcher das Mesylat-Salz von U.K. 52 046 verwendet wird.

## Revendications

**1.** Utilisation du composé U.K.-52 046 qui répond à la formule chimique :

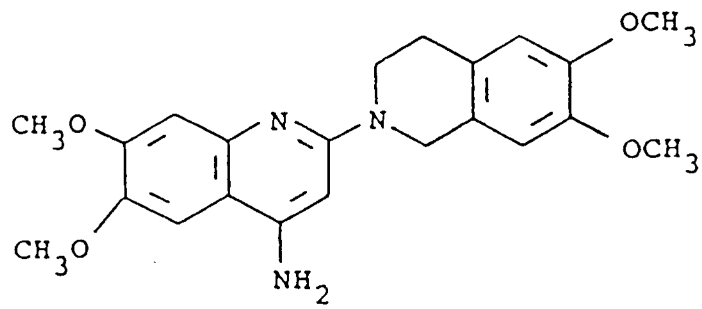

ou d'un sel d'addition d'acide pharmaceutiquement acceptable de ce composé, pour l'obtention d'un médicament destiné à traiter l'impuissance érectile chez l'homme.

**2.** Utilisation du composé U.K.-52 046 ou d'un sel pharmaceutiquement acceptable de ce composé suivant la revendication 1, dans laquelle le médicament convient pour l'administration parentérale.

**3.** Utilisation du composé U.K.-52 046 ou d'un sel pharmaceutiquement acceptable de ce composé suivant la revendication 1, dans laquelle le médicament convient pour l'administration transdermique.

**4.** Utilisation suivant la revendication 2 ou 3, dans laquelle le sel de U.K.-52 046 utilisé est le mésylate.